# EUROPEAN PATENT SPECIFICATION

(11) **EP 4 171 692 B1**
(45) Date of publication and mention of the grant of the patent: **22.10.2025**
(21) Application number: 21732935.8
(22) Date of filing: 22.06.2021
(51) Int. Cl.: A61M 15/06, A24F 40/42, A61M 11/04, A24F 40/465, A24F 40/60

(54) **AEROSOL-GENERATING DEVICE WITH ILLUMINATED PROXIMAL END**
AEROSOLERZEUGENDE VORRICHTUNG MIT BELEUCHTETEM PROXIMALEM ENDE
DISPOSITIF DE GÉNÉRATION D'AÉROSOL COMPORTANT UNE EXTRÉMITÉ PROXIMALE ÉCLAIRÉE

(30) Priority: 25.06.2020 EP 20182259
(43) Date of publication of application: 03.05.2023
(73) Proprietor: Philip Morris Products S.A., 2000 Neuchâtel (CH)
(72) Inventor: BATISTA, Rui Nuno, 2000 Neuchâtel (CH); CALI, Ricardo, 68163 Mannheim (DE)
(74) Representative: Abitz & Partner
(86) International application number: PCT/EP2021/067036
(87) International publication number: WO 2021/259949

(56) References cited:
- US-A1- 2015 238 713
- US-A1- 2015 305 409
- US-A1- 2019 231 997

## Description

The present disclosure relates to an aerosol-generating device with an elongate housing having a distal end and a proximal end, with a light source, and a control unit configured for controlling the light source. The proximal end of the housing comprises an illumination portion made from translucent material and the light source is optically coupled to the illumination portion. The present disclosure also relates to an aerosol-generating system comprising the aerosol-generating device and a method for operating the aerosol-generating system.

Visual indication means are frequently used in electronic aerosol-generating devices to visually provide information to the user. Existing aerosol-generating devices comprising different types of displays in their lateral surfaces are known in the art.

Currently used visual indication means are provided at positions of the aerosol-generating devices that cannot be easily observed during the user experience. Since such visual indication means are usually provided at the sides of the housing of the aerosol-generating device, the aerosol-generating device may have to be turned around to be inspected. Such handling may be perceived as cumbersome by the consumers.

It is also known to provide end faces that in use point away from the consumer with illuminated portions. Typically, such illuminated portions are used in order to mimic the glow of a conventional cigarette.

It would be desirable to provide an aerosol-generating device having a visual feedback means that can conveniently be checked during the user experience.

It would further be desirable to provide an aerosol-generating device having a visual feedback means that may guide the user before, during and after the user experience.

It would be further desirable to provide an aerosol-generating device with a feedback means that has a low power consumption and at the same time has a high visual perceptibility.

Patent document US2019/231997A1 is hereby acknowledged.

According to an embodiment of the present disclosure there is provided an aerosol-generating device comprising an elongate housing having a distal end and a proximal end, a light source, and a control unit configured for controlling the light source. The proximal end of the housing comprises an illumination portion made from translucent material. The light source is optically coupled to the illumination portion.The aerosol-generating device may be configured to receive an aerosol-generating article. The illumination portion may be configured to surround the aerosol-generating article in use.

As used herein, an 'aerosol-generating device' relates to a device that interacts with an aerosol-forming substrate to generate an aerosol. The aerosol-forming substrate may be part of an aerosol-generating article. An aerosol-generating device may be a device that interacts with an aerosol-forming substrate of an aerosol-generating article. An aerosol-generating device may be a holder. The aerosol-generating device may be an electrically heated aerosol-generating device. The aerosol-generating device may comprise one or more of a housing, electric circuitry, a power supply, a heating chamber and a heating element. The housing may define a cavity that comprises the heating chamber having an open end at the proximal end of the aerosol-generating device, and the heating element. The heating chamber may be configured for insertion of an aerosol-generating article. The heating element may be part of the aerosol-generating article.

The aerosol-generating device is preferably a portable or handheld device that is comfortable to hold between the fingers of a single hand. The device may be substantially cylindrical in shape and may have a length of between 30 and 150 millimetres. The maximum diameter of the device is preferably between 5 and 30 millimetres.

The housing of the aerosol-generating device may be elongate. The housing may comprise any suitable material or combination of materials. Examples of suitable materials include metals, alloys, plastics or composite materials containing one or more of those materials, or thermoplastics that are suitable for food or pharmaceutical applications, for example polypropylene, polyetheretherketone (PEEK) and polyethylene. Preferably, the material is light and non-brittle.

The housing may comprise a mouthpiece. The housing may comprise at least one air inlet. The housing may comprise more than one air inlet. The mouthpiece may comprise at least one air inlet and at least one air outlet. The mouthpiece may comprise more than one air inlet. One or more of the air inlets may reduce the temperature of the aerosol before it is delivered to a user and may reduce the concentration of the aerosol before it is delivered to a user.

As used herein, the term 'mouthpiece' refers to a portion of an aerosol-generating device that during a puff is closest to the user's mouth.

The aerosol-generating device may include a sensor to activate the aerosol-generating device. The aerosol-generating device may include a user interface to activate the aerosol-generating device, for example a button to initiate heating of the aerosol-generating device or display to indicate a state of the aerosol-generating device or of the aerosol-forming substrate.

As used herein, the term 'user experience' refers to the act of consuming an aerosol-generating article. A user experience may include one or more consecutive puffs. The user experience also includes the time period in between puffs.

As light source of the aerosol-generating device any known light source with low power consumption may be used. Suitable light sources include light emitting diodes (LED), micro LEDs or organic LEDs (OLED) devices. These light sources have low power consumption. The addition of a the light source with low power consumption advantageously puts only a very small load on the power source of an existing aerosol-generating device, preferably with only very limited effect on the design of the existing power source. This is particularly advantageous for portable devices in which capacity of the power source is limited and excess power consumption is to be avoided.

The light source may be capable of displaying monochromatic or multi-coloured light. A light source that is capable of displaying multi-coloured light may be controlled to emit a plurality of colours of light. Multi-coloured light sources are advantageous in that a single light source can be used to create a plurality of illumination effects. This may help to reduce the overall number of electronic components to be used in the aerosol-generating device. This may also reduce design complexity and may reduce the total manufacturing cost of the aerosol-generating device.

In use of the aerosol-generating device the proximal end of the housing, which comprises the illumination portion, may point towards the consumer. The housing of the aerosol-generating device may define a cavity with an open end at the proximal end of the housing. The cavity may be configured to receive an aerosol-generating article.

The proximal end of the housing is the end at which the mouthpiece is provided. During a puff the user puts the mouthpiece into or close to the user's mouth.

The proximal end may have a proximal end face that faces to the user during the user experience. The proximal end face may comprise the illumination portion.

Accordingly, in particular in use of the aerosol-generating device, the proximal end of the housing and under normal handling conditions the proximal end of the aerosol-generating device is in the field of view of the user. The illumination portion provided at the proximal end of the aerosol-generating device is therefore highly visible to the user in use of the device and also during the user experience. The illumination portion is particularly visible to the user in between puffs of a user experience.

For this reason, the illumination portion may be advantageously used to indicate useful information to the user. Such information may pertain to the various operation status of the aerosol-generation device. The illumination portion may also be used to indicate a progress of a specific operation or information regarding the user experience.

The aerosol-generating device may comprise a retaining element. The retaining element may form the illumination portion at the proximal end of the housing of the aerosol-generating device. The retaining element may be provided at the opening of the cavity formed at the proximal end of the housing of the aerosol-generating device.

The retaining element may have generally ring-shaped form. The outer diameter of the retaining element may correspond to the dimensions of the housing of the aerosol-generating device. The outer circumferential edge of the retaining element may be mounted to the housing of the aerosol-generating device.

The inner diameter of the retaining element may define the opening of the cavity. The inner diameter of the retaining element may have a dimension that corresponds to the diameter of the aerosol-generating article that is to be inserted into the cavity. The retaining element may assure correct placement of the aerosol-generating article in the cavity. In particular, the retaining element may ensure that the aerosol-generating article is correctly inserted in the centre of the cavity.

The retaining element may be made from any suitable material. The retaining element may be made from an elastic material. The retaining element is made from elastic, translucent polymer material such as, for example, poly(amide-imide), liquid silicone rubber (LSR), polyvinyl chloride (PVC). Retaining elements made from such elastic materials may be dimensioned such that their inner diameter corresponds exactly or is slightly smaller than the outer diameter of the aerosol-generating article that is to be inserted into the cavity. The retaining element may be formed such that it has physical contact with the outer circumference of the aerosol-generating article that is inserted into the cavity. In this way the retaining element may assure retention of the aerosol-generating article in the cavity. Moreover, the retaining element may sealingly engage with the outer circumference of the aerosol-generating article in the cavity. In use of the aerosol-generating device the retaining element may reduce air flow into the heating chamber through its open end.

The retaining element may be made from transparent or translucent material. The retaining element may be optically coupled to the light source of the aerosol-generating device. By coupling the retaining element optically to the light source, the retaining element may be illuminated by the light source. When the light source is configured to generate a plurality of different colours, the retaining element can accordingly appear in a number of different colours.

The light source can be integrated in the opening of the cavity of the device and in close proximity of the retaining element. The light source may be provided in a circumferential recess at the opening of the cavity. The light source may have a ring-shaped form that may be integrated in the opening of the cavity of the device and in close proximity of the retaining element. The light source may be located directly adjacent to the retaining element. The light source may be located directly adjacent to the outer circumference of the retaining element. By providing the light source close to the translucent retaining element, the light can be efficiently coupled into the material of the retaining element.

The aerosol-generating device may be an electrically heated aerosol-generating device. The aerosol-generating device may be configured to resistively heat the aerosol-generating article.

The aerosol-generating device may be configured to inductively heat the aerosol-generating article. The aerosol-generating device may comprise an induction heating element. The induction heating element may comprise an induction coil. The induction heating element may comprise a susceptor. The induction coil is capable of generating an alternating magnetic field. This alternating magnetic field penetrates the susceptor and thereby generates heat due to eddy currents and or hysteresis losses within the susceptor.

The susceptor may be formed from any material that can be inductively heated to a temperature sufficient to generate an aerosol from the aerosol-forming substrate. A preferred susceptor may comprise or consist of a ferromagnetic material or ferri-magnetic material, for example a ferromagnetic alloy, ferritic iron, or a ferromagnetic steel or stainless steel. A suitable susceptor may be, or comprise, aluminum. Preferred susceptors may be heated to a temperature in excess of 250 degrees Celsius.

When an induction heating element is employed, the induction heating element may be configured as an internal heating element as described herein or as an external heater as described herein. If the induction heating element is configured as an internal heating element, the susceptor element is preferably configured as a pin or blade for penetrating the aerosol-generating article. If the induction heating element is configured as an external heating element, the susceptor element is preferably configured as a cylindrical susceptor at least partly surrounding the cavity or forming the sidewall of the cavity.

The susceptor may also be provided within or as a part of the aerosol-forming article.

The aerosol-generating device comprises a control unit that is configured to control the operation of the aerosol-generating device. The control unit may be configured to control the heating element of the aerosol-generating device.

The control unit may be configured to control the light source of the aerosol-generating device. The control unit may be configured to control the light source of the aerosol-generating device based on the detected operation status of the aerosol-generating device.

Controlling the light source based on an operation status of the aerosol-generating device may include changing colour, brightness or operational mode of the light source. The operational mode of the light source may include continuous light mode, blinking mode or flashing mode.

The control unit may detect activation of the aerosol-generating device and may be configured to control the light source to emit a first colour of light to indicate that the aerosol-generating device is activated. The control unit may control the light source to emit white light to indicate that the aerosol-generating device is activated.

The control unit may detect that heat-up procedure is started and may be configured to control the light source to emit a second colour of light to indicate that the aerosol-generating device is heating up. The control unit may be configured to control the light source to blink in the second colour to indicate that the process of heating up is currently carried out.

The control unit may detect that heat-up procedure has finished and that the aerosol-generating device is ready for the user experience. The control unit may control the light source to emit a further colour of light to indicate that the aerosol-generating device is ready for use. The control unit may control the light source to emit a green colour to indicate that the aerosol-generating device is ready for use.

By controlling the light source to changing its lighting status and thereby changing the appearance of the illumination portion, the user can be informed of the operational status of the aerosol-generating device. Since the illumination portion is provided at the proximal end of the housing of the aerosol-generating device, the illumination portion is readily visible to the user during the user experience. Furthermore the illumination portion at the proximal end of the housing of the aerosol-generating device during normal handling of the device is usually not covered by a user's hand, such that the risk of accidentally covering the illumination portion is reduced.

The control unit of the aerosol-generating device may be configured to control the light source also based on signals detected from further sensors of the aerosol-generating device.

The proximal end of the aerosol-generating device may comprise a proximity sensor for detecting proximity to the consumer's mouth. Based on corresponding signals received from the proximity sensor, the light source may be controlled by the control unit. The control unit may dim the light source during a puff, when the proximity sensor detects proximity to the consumer's mouth. During a puff the user may not be able to observe the proximal end of the aerosol-generating device. In such situation the light source can be temporarily dimmed or switched off to save electric energy of the power source.

The aerosol-generating device may comprise a light sensor for detecting the brightness of the ambient environment. The control unit may be configured to adjust the intensity of the light source based on the signals from the light sensor. In this way it can be ensured that during the day at increase surrounding brightness the intensity of the light source is sufficient to be detected by the user. At the same time electric energy may be saved by avoiding unnecessary power consumption by a high intensity of the light source at night or in low light environment.

The control unit of the aerosol-generating device may be configured to detect the presence of an aerosol-generating article in the cavity. For this purpose the control unit may be configured to monitor the magnetic field within the heating chamber of the aerosol-generating device. In embodiments in which the aerosol-generating article is inductively heated the change of the magnetic field of the induction coils may be monitored. The control unit may not only determine whether a change of the magnetic field occurs, but may also be configured to determine whether the aerosol-generating article has been inserted correctly. The control unit may also be configured to determine whether the correct aerosol-generating article has been inserted. Aerosol-generating devices are usually configured to co-operate with specific aerosol-generating articles. Thus, a recognition method that is able to verify that the correct aerosol-generating articles are used with the device may help to avoid incorrect use of the aerosol-generating device.

The control unit may be configured to control the light source in dependence of the aerosol-generating article recognition. If the control unit detects that a compatible aerosol-generating article has been correctly inserted into the heating chamber of the aerosol-generating device, the light source may be controlled to change to a further different colour to indicate to the user that the aerosol-generating device is ready for use. The control unit may also be configured to automatically start heating when a correct aerosol-generating article has been correctly inserted into the heating chamber of the aerosol-generating device.

The present technology is also directed to an aerosol-generating system comprising an aerosol-generating device as described above and an aerosol-generating article configured to be inserted into the cavity of the aerosol-generating device, wherein the aerosol-generating device comprise an elongate housing having a distal end and a proximal end, a light source, and a control unit configured for controlling the light source. The proximal end of the housing comprises an illumination portion made from translucent material, and the light source is optically coupled to the illumination portion. The control unit is configured to control the light source based on an operation status of the aerosol-generating device.

The present technology is also directed to a method of operating an aerosol-generating system comprising an aerosol-generating device as described above and an aerosol-generating article configured to be inserted into the cavity of the aerosol-generating device. The control unit controls the light source based on an operation status of the aerosol-generating device.

Controlling the light source based on an operation status of the aerosol-generating device may include changing colour, brightness or the lighting mode of the light source. The operational mode of the light source may include continuous light mode, blinking mode or flashing mode.

The control unit may be configured to detect presence of an aerosol-generating article in the cavity and may changes brightness, colour or operational mode of the light source upon detection of the presence of the aerosol-generating article.

The control unit may be configured to detect presence of an aerosol-generating article in the cavity by monitoring the magnetic field in the heating chamber. This method is particularly advantageous for aerosol-generating devices that are configured for inductive heating. Such devices are configured to generate a magnetic field in the heating chamber, such that monitoring of the magnetic field can be performed without increasing the complexity of the aerosol-generating device.

The aerosol-generating system may be operated such that the heat-up process is automatically started upon insertion of an aerosol-generating article into the heating chamber of the aerosol-generating device.

The control unit may be configured to detect the operation temperature of the aerosol-generating device. The control unit may be configured to adjust the light source based on the detected temperature of the heating chamber. In this way the user may be visually informed by inspection of the light source that the aerosol-generating system is ready for use.

The control unit may be configured to control the light source to emit a visual warning signal in case one of the signals received at the control unit are outside an expected range. In this way the user may be immediately informed should an unexpected situation be detected. Such situation may include for example the detection of an incompatible aerosol-generating article, incorrect insertion of a compatible aerosol-generating article, or detection of an imminent overheating of the heating chamber. In such case, the control unit may switch the light source into a flashing mode. Since the illumination portion is usually within the view field of the user, the user may immediately perceive the warning signal.

The aerosol generating device may be configured to perform a cleaning procedure of the heating chamber. The cleaning procedure may include heating the heating chamber to increased temperatures. The control unit may be configured to control the light source to emit a corresponding visual signal to inform the user that a cleaning procedure is in operation.

Thanks to the good visibility of the illumination portion at the proximal end of the aerosol-generating device, the user is guided and informed before, during and after the user experience. In this way the overall user experience is enhanced.

A user may be able to customize the aerosol-generating device by adapting the light control according to personal preferences. Customization may be done by connecting the aerosol generating device to a personal computer, a smart phone or any other suitable input device. By customizing the aerosol-generating device, the user may choose how the light source is controlled by the control unit. For example, the user may select colour, brightness, blinking frequencies, or colour sequences of the light source.

The invention will be further described, by way of example only, with reference to the accompanying drawings in which:
Fig. 1 shows an aerosol-generating system; and
Fig. 2 shows cross-sectional views of the heating chamber of an aerosol-generating device.

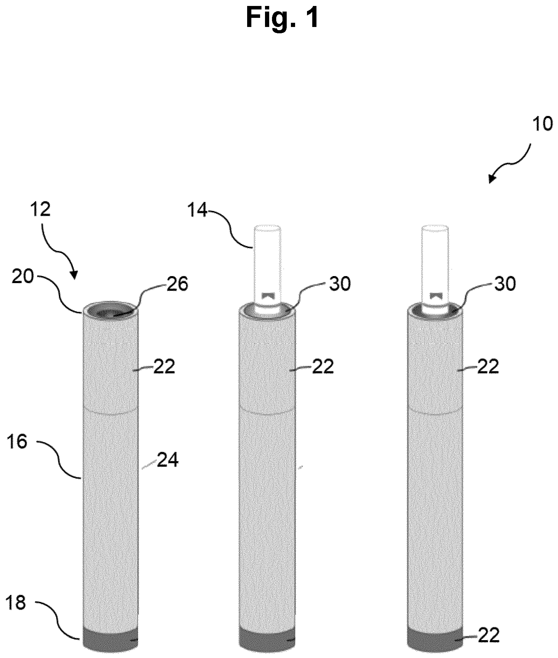

In Fig. 1 an aerosol-generating system 10 comprising an aerosol-generating device 12 and an aerosol-generating article 14 are depicted. The aerosol-generating device 12 comprises an elongate housing 16 having a distal end 18 and a proximal end 20. The housing defines a main portion 22 and a heating portion 24. In the main portion 22 of the housing 16 of the aerosol-generating device 12 there is provided a power source and electric circuitry including a control unit. The power source provides electrical energy to the control unit and all other electronic components of the aerosol-generating device 12. An interface portion 22 is located at the distal end 18 of the housing 16. The interface portion 22 comprises a charging port for charging the power source.

The heating portion 24 of the housing 16 defines a cavity 26 at the proximal end 20. The cavity 26 forms a heating chamber that is configured for receiving and heating the aerosol-generating article 14. At the opening of the cavity 26 at the proximal end 20 of the housing 16 there is provided an illumination portion 30. The illumination portion 30 is made from translucent material. The illumination portion 30 is coupled to a multi-colour light source and is configured to be illuminated in a plurality of colours.

In the left view in Fig. 1 the aerosol-generating device 12 is activated and ready for receiving an aerosol-generating article 14. As an indication of this operational status, the illumination portion 30 is illuminated in bright blue colour.

In the central view in Fig. 1 the aerosol-generating article 14 is inserted into the cavity 26 of the aerosol-generating device 12 and the heating phase is started. As an indication of this operational status, the illumination portion 30 is illuminated in red colour.

In the right view in Fig. 1 the heating phase is finished and the aerosol-generating system 10 is ready for the user experience. As an indication of this operational status, the illumination portion 30 is illuminated in green colour.

A user usually holds the aerosol-generating device 12 at the central portion of the housing 16, leaving the proximal end 20 uncovered. In normal use of the aerosol-generating device 12 the proximal end 20 points towards the user. Accordingly, the proximal end 20 is usually in the view field of the user and the information conveyed by the colour coding of the illumination portion 30 is readily perceivable by the user.

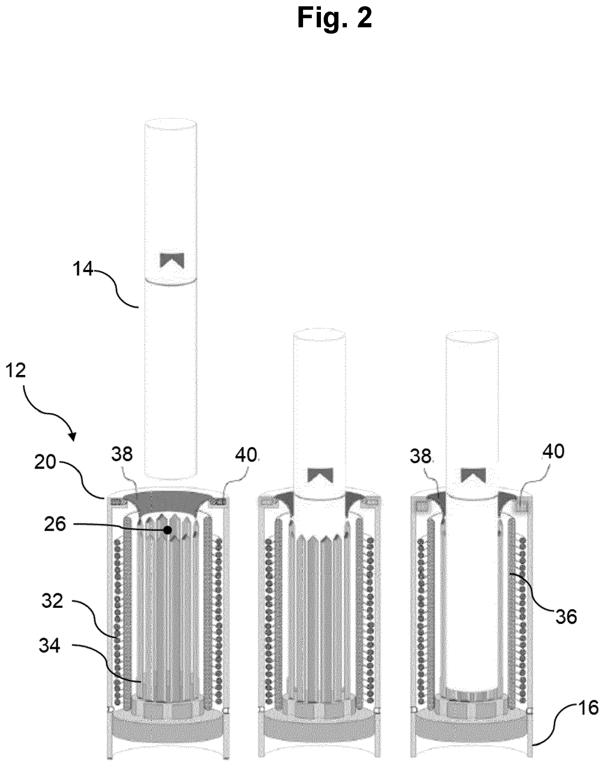

In the cross-sectional views of Fig. 2 more details of the heating chamber can be seen. In this embodiment the aerosol-generating device 12 is configured for inductive heating. For this purpose the aerosol-generating device 12 comprises an induction coil 32 and a susceptor element 34. The induction coil 32 and the susceptor element 34 are separated by an electrically insulating wall 36 and are arranged to surround the cavity of aerosol-generating device 12.

The induction coil 32 is capable of generating an alternating magnetic field. This alternating magnetic field penetrates the susceptor element 34 and thereby generates heat.

The susceptor element 34 has a cylindrical shape and has a diameter that substantially corresponds to the outer diameter of the aerosol-generating article 14. The susceptor element 34 is formed from a plurality of susceptor blades. The susceptor blades are configured resilient and firmly contact the aerosol-generating article 14 after insertion. In this way good thermal contact between the susceptor element 34 and the aerosol-generating article 24 is ensured.

The opening of the cavity 26 at the proximal end 20 of the housing 16 there is provided a ring shaped retaining element 38 made from elastic and translucent polymer material. In this case the retaining element 38 is made from polyvinyl chloride. The retaining element forms the illumination portion of the aerosol-generating device 12. The retaining element 38 defines the opening of the cavity 26 and has an inner diameter that corresponds to the outer diameter aerosol-generating article 14. The funnel shape of the retaining element 38 allows for guided and easy insertion of the aerosol-generating article 14 into the heating chamber.

A light source 40 is optically coupled to the retaining element 38. The light source 40 is an OLED device that is capable of emitting a plurality of colours. The light source 40 is located in a circumferential recess in the housing 16 adjacent to the outer circumference of the retaining element 38. Since the retaining element 38 is made from translucent material, the retaining element 38 appears in the colour that is emitted from the light source.

When the aerosol-generating device 12 is turned off, the light source 40 is deactivated, and the retaining element 30 appears in the natural colour of its material. When the aerosol-generating device 12 is activated, but an aerosol-generating article 14 is not yet inserted, the light source 40 is controlled to emit bright blue light, to inform the user that the aerosol-generating device 12 is activated.

In the embodiment of Fig. 2, the aerosol-generating device 12 is configured for monitoring the magnetic field within the heating chamber. If the aerosol-generating article 14 is inserted into the heating chamber, the control unit detects a change of the magnetic field. If the change of the magnetic field corresponds to an expected change upon correct insertion of a compatible aerosol-generating article 14, the light source 40 is switched to emit green pulsing light. This optical feedback confirms the user of correct handling of the aerosol-generating system 10.

The control unit is further configured to automatically start the heating phase after a designated time of three seconds after insertion of the aerosol-generating article 14. During the heating phase the light source 40 is switched to emit orange pulsing light.

When the heating phase is completed, the control unit switches the colour of the light source 40 from orange pulsing to a continuous green light to inform the user that the aerosol-generating system 10 is ready for the user experience.

If the control unit of the aerosol-generating device 12 detects upon monitoring of the magnetic field, that an aerosol-generating article 14 is wrongly inserted or that an incompatible aerosol-generating article 14 is inserted, the control unit controls the light source 40 to emit red flashing light. At the same time normal operation of aerosol-generating system 10 is interrupted until correct insertion of the aerosol-generating article 14 is established.

## Claims

1. Aerosol-generating device (12) configured to receive an aerosol-generating article (14), comprising
- an elongate housing (16) having a distal end (18) and a proximal end (20),
- a light source (40), and
- a control unit configured for controlling the light source (40),
wherein in use of the aerosol-generating device (12) the proximal end (20) of the housing (16) points towards the consumer,
wherein the proximal end (20) of the housing (16) comprises an illumination portion (30) made from translucent material, and wherein the light source (40) is optically coupled to the illumination portion (30), and wherein the illumination portion (30) is configured to surround the aerosol-generating article (14) in use.

2. Aerosol-generating device (12) according to claim 1, wherein the light source (40) is a LED, micro LED or OLED.

3. Aerosol-generating device (12) according to any one of the preceding claims, wherein the light source (40) is capable of displaying monochromatic or multi-coloured light.

4. Aerosol-generating device (12) according to any one of the preceding claims, wherein the housing (16) of the aerosol-generating device (12) defines a cavity (26) with an open end at the proximal end (20) of the housing (16), and wherein the cavity (26) is configured to receive the aerosol-generating article (14).

5. Aerosol-generating device (12) according to any one of the preceding claims, wherein the aerosol-generating device (12) comprises a retaining element (38) and wherein the retaining element (38) forms the illumination portion (30) at the proximal end (20) of the housing (16).

6. Aerosol-generating device (12) according to claim 5, wherein the retaining element (38) is made from elastic material.

7. Aerosol-generating device (12) according to any one of the preceding claims, wherein the control unit is configured to control the light source (40) based on the detected operation status of the aerosol-generating device (12).

8. Aerosol-generating system comprising an aerosol-generating device (12) according to any one of the preceding claims, and the aerosol-generating article (14) configured to be inserted into the cavity (26) of the aerosol-generating device (12).

9. Method of operating an aerosol-generating system according to claim 8, wherein the control unit is configured to control the light source (40) based on an operation status of the aerosol-generating device (12).

10. Method of operating an aerosol-generating system according to claim 9, wherein the control unit is configured to detect a change of the operation status of the aerosol-generating device (12), and wherein the control unit changes brightness, the colour or the lighting mode of the light source (40) to indicate the change of the operation status.

11. Method of operating an aerosol-generating system according to claims 9 or 10, wherein the control unit is configured to detect presence of a consumable and wherein the control unit changes brightness and or the colour of the light source (40) upon detection of the presence of the consumable.

12. Method of operating an aerosol-generating system according to claims 9 to 11, wherein the aerosol generating device is configured to inductively heat the consumable, and wherein the control unit is configured to detect presence of a consumable by monitoring the magnetic field in the heating chamber.

13. Method of operating an aerosol-generating system according to claims 9 to 12, wherein the control unit is configured to emit a visual warning signal if a monitored parameter is without an expected range.

14. Method of operating an aerosol-generating system according to claims 9 to 13, wherein aerosol generating device is configured to perform a cleaning procedure, and wherein the control unit is configured to control the light source (40) to emit a corresponding visual signal.

## Patentansprüche

1. Aerosolerzeugungsvorrichtung (12), die eingerichtet ist, einen aerosolerzeugenden Artikel (14) aufzunehmen, aufweisend
- ein längliches Gehäuse (16) mit einem distalen Ende (18) und einem proximalen Ende (20),
- eine Lichtquelle (40), und
- ein Steuergerät, das dazu eingerichtet ist, die Lichtquelle (40) zu regeln,
wobei bei Gebrauch der Aerosolerzeugungsvorrichtung (12) das proximale Ende (20) des Gehäuses (16) in Richtung des Verbrauchers zeigt,
wobei das proximale Ende (20) des Gehäuses (16) einen Beleuchtungsabschnitt (30) aufweist, der aus lichtdurchlässigem Material hergestellt ist, und wobei die Lichtquelle (40) optisch mit dem Beleuchtungsabschnitt (30) gekoppelt ist, und wobei der Beleuchtungsabschnitt (30) dazu eingerichtet ist, den aerosolerzeugenden Artikel (14) bei Gebrauch zu umgeben.

2. Aerosolerzeugungsvorrichtung (12) nach Anspruch 1, wobei die Lichtquelle (40) eine LED, Mikro-LED oder OLED ist.

3. Aerosolerzeugungsvorrichtung (12) nach einem beliebigen der vorhergehenden Ansprüche, wobei die Lichtquelle (40) in der Lage ist, monochromatisches oder mehrfarbiges Licht anzuzeigen.

4. Aerosolerzeugungsvorrichtung (12) nach einem beliebigen der vorhergehenden Ansprüche, wobei das Gehäuse (16) der Aerosolerzeugungsvorrichtung (12) einen Hohlraum (26) mit einem offenen Ende an dem proximalen Ende (20) des Gehäuses (16) definiert, und wobei der Hohlraum (26) dazu eingerichtet ist, den aerosolerzeugenden Artikel (14) aufzunehmen.

5. Aerosolerzeugungsvorrichtung (12) nach einem beliebigen der vorhergehenden Ansprüche, wobei die Aerosolerzeugungsvorrichtung (12) ein Rückhalteelement (38) aufweist und wobei das Rückhalteelement (38) den Beleuchtungsabschnitt (30) an dem proximalen Ende (20) des Gehäuses (16) bildet.

6. Aerosolerzeugungsvorrichtung (12) nach Anspruch 5, wobei das Rückhalteelement (38) aus elastischem Material hergestellt ist.

7. Aerosolerzeugungsvorrichtung (12) nach einem beliebigen der vorhergehenden Ansprüche, wobei das Steuergerät dazu eingerichtet ist, die Lichtquelle (40) basierend auf dem detektierten Betriebszustand der Aerosolerzeugungsvorrichtung (12) zu regeln.

8. Aerosolerzeugungssystem, aufweisend eine Aerosolerzeugungsvorrichtung (12) nach einem beliebigen der vorhergehenden Ansprüche, und den aerosolerzeugenden Artikel (14), der dazu eingerichtet ist, in den Hohlraum (26) der Aerosolerzeugungsvorrichtung (12) eingesetzt zu werden.

9. Verfahren für ein Betreiben eines Aerosolerzeugungssystems nach Anspruch 8, wobei das Steuergerät dazu eingerichtet ist, die Lichtquelle (40) basierend auf einem Betriebszustand der Aerosolerzeugungsvorrichtung (12) zu regeln.

10. Verfahren für ein Betreiben eines Aerosolerzeugungssystems nach Anspruch 9, wobei das Steuergerät dazu eingerichtet ist, eine Änderung des Betriebszustands der Aerosolerzeugungsvorrichtung (12) zu detektieren, und wobei das Steuergerät die Helligkeit, die Farbe oder den Beleuchtungsmodus der Lichtquelle (40) ändert, um die Änderung des Betriebszustands anzuzeigen.

11. Verfahren für ein Betreiben eines Aerosolerzeugungssystems nach Anspruch 9 oder 10, wobei das Steuergerät dazu eingerichtet ist, das Vorhandensein eines Verbrauchsartikels zu detektieren und wobei das Steuergerät die Helligkeit und/oder die Farbe der Lichtquelle (40) bei Detektion des Vorhandenseins des Verbrauchsartikels ändert.

12. Verfahren für ein Betreiben eines Aerosolerzeugungssystems nach einem der Ansprüche 9 bis 11, wobei die Aerosolerzeugungsvorrichtung dazu eingerichtet ist, den Verbrauchsartikel induktiv zu erwärmen, und wobei das Steuergerät dazu eingerichtet ist, das Vorhandensein eines Verbrauchsartikels durch Überwachen des Magnetfeldes in der Heizkammer zu detektieren.

13. Verfahren für ein Betreiben eines Aerosolerzeugungssystems nach einem der Ansprüche 9 bis 12, wobei das Steuergerät dazu eingerichtet ist, ein optisches Warnsignal auszugeben, wenn ein überwachter Parameter außerhalb eines erwarteten Bereichs liegt.

14. Verfahren für ein Betreiben eines Aerosolerzeugungssystems nach einem der Ansprüche 9 bis 13, wobei die Aerosolerzeugungsvorrichtung dazu eingerichtet ist, einen Reinigungsvorgang durchzuführen, und wobei das Steuergerät dazu eingerichtet ist, die Lichtquelle (40) zu regeln, um ein entsprechendes visuelles Signal auszugeben.

## Revendications

1. Dispositif de génération d'aérosol (12) configuré pour recevoir l'article de génération d'aérosol (14), comprenant
- un logement allongé (16) ayant une extrémité distale (18) et une extrémité proximale (20),
- une source lumineuse (40), et
- une unité de commande configurée pour commander la source lumineuse (40),
dans lequel, lors de l'utilisation du dispositif de génération d'aérosol (12), l'extrémité proximale (20) du logement (16) est orientée vers le consommateur,
dans lequel l'extrémité proximale (20) du logement (16) comprend une portion d'éclairage (30) composée d'un matériau translucide, et dans lequel la source lumineuse (40) est couplée optiquement à la portion d'éclairage (30), et dans lequel la portion d'éclairage (30) est configurée pour entourer l'article de génération d'aérosol (14) en utilisation.

2. Dispositif de génération d'aérosol (12) selon la revendication 1, dans lequel la source lumineuse (40) est une DEL, une micro-LED ou une OLED.

3. Dispositif de génération d'aérosol (12) selon l'une quelconque des revendications précédentes, dans lequel la source lumineuse (40) est capable d'afficher une lumière monochrome ou multicolore.

4. Dispositif de génération d'aérosol (12) selon l'une quelconque des revendications précédentes, dans lequel le logement (16) du dispositif de génération d'aérosol (12) définit une cavité (26) avec une extrémité ouverte au niveau de l'extrémité proximale (20) du logement (16), et dans lequel la cavité (26) est configurée pour recevoir l'article de génération d'aérosol (14).

5. Dispositif de génération d'aérosol (12) selon l'une quelconque des revendications précédentes, dans lequel le dispositif de génération d'aérosol (12) comprend un élément de retenue (38) et dans lequel l'élément de retenue (38) forme la portion d'éclairage (30) au niveau de l'extrémité proximale (20) du logement (16).

6. Dispositif de génération d'aérosol (12) selon la revendication 5, dans lequel l'élément de retenue (38) est composé d'un matériau élastique.

7. Dispositif de génération d'aérosol (12) selon l'une quelconque des revendications précédentes, dans lequel l'unité de commande est configurée pour commander la source lumineuse (40) sur la base de l'état de fonctionnement détecté du dispositif de génération d'aérosol (12).

8. Système de génération d'aérosol comprenant un dispositif de génération d'aérosol (12) selon l'une quelconque des revendications précédentes, et l'article de génération d'aérosol (14) configuré pour être inséré dans la cavité (26) du dispositif de génération d'aérosol (12).

9. Procédé de fonctionnement d'un système de génération d'aérosol selon la revendication 8, dans lequel l'unité de commande est configurée pour commander la source lumineuse (40) sur la base d'un état de fonctionnement du dispositif de génération d'aérosol (12).

10. Procédé de fonctionnement d'un système de génération d'aérosol selon la revendication 9, dans lequel l'unité de commande est configurée pour détecter un changement de l'état de fonctionnement du dispositif de génération d'aérosol (12), et dans lequel l'unité de commande change la luminosité, la couleur ou le mode d'éclairage de la source lumineuse (40) pour indiquer le changement de l'état de fonctionnement.

11. Procédé de fonctionnement d'un système de génération d'aérosol selon les revendications 9 ou 10, dans lequel l'unité de commande est configurée pour détecter la présence d'un consommable et dans lequel l'unité de commande change la luminosité et/ou la couleur de la source lumineuse (40) lors de la détection de la présence du consommable.

12. Procédé de fonctionnement d'un système de génération d'aérosol selon les revendications 9 à 11, dans lequel le dispositif de génération d'aérosol est configuré pour chauffer par induction le consommable, et dans lequel l'unité de commande est configurée pour détecter la présence d'un consommable en surveillant le champ magnétique dans la chambre de chauffage.

13. Procédé de fonctionnement d'un système de génération d'aérosol selon les revendications 9 à 12, dans lequel l'unité de commande est configurée pour émettre un signal d'avertissement visuel si un paramètre surveillé est dépourvu d'une plage attendue.

14. Procédé de fonctionnement d'un système de génération d'aérosol selon les revendications 9 à 13, dans lequel le dispositif de génération d'aérosol est configuré pour réaliser une procédure de nettoyage, et dans lequel l'unité de commande est configurée pour commander la source lumineuse (40) pour émettre un signal visuel correspondant.
